Europäisches Patentamt

**European Patent Office**

Office européen des brevets

(11) Publication number: **0 089 136**
**A2**

(12) # EUROPEAN PATENT APPLICATION

(21) Application number: **83301050.7**

(22) Date of filing: **28.02.83**

(51) Int. Cl.³: **A 61 K 7/16**
**A 61 K 7/22, A 61 K 7/24**
**A 61 K 7/18**

(30) Priority: **10.03.82 US 356678**
**03.02.83 US 463378**

(43) Date of publication of application:
**21.09.83 Bulletin 83/38**

(84) Designated Contracting States:
**AT BE CH DE FR GB IT LI NL SE**

(71) Applicant: **THE PROCTER & GAMBLE COMPANY**
**301 East Sixth Street**
**Cincinnati Ohio 45201(US)**

(72) Inventor: **Mobley, Michael Joseph**
**6182 Delcrest Ct.**
**Fairfield Ohio 45014(US)**

(74) Representative: **Brooks, Maxim Courtney et al,**
**Procter & Gamble (NTC) Limited Whitley Road**
**Longbenton**
**Newcastle-upon-Tyne NE12 9TS(GB)**

(54) Oral hygiene products.

(57) Oral hygiene products comprising of a calcium ion source, a fluoride ion source, and a calcium sequestering agent, having specific binding and solubility characteristics, provide effective anticaries protection.

EP 0 089 136 A2

Croydon Printing Company Ltd.

0089136

# ORAL HYGIENE PRODUCTS

## MICHAEL J. MOBLEY

This invention relates to oral hygiene products providing anticaries protection to teeth. The products comprise a calcium ion source, a fluoride ion source and a calcium sequestering agent having specific binding and solubility characteristics to control the precipitation of $CaF_2$.

There are several mechanisms through which fluoride is thought to exert its anticaries effect. One possible mechanism involves fluoride's antimicrobial effects on oral bacteria. One antimicrobial effect seems to center around a reduction in the acidic production of plaque. Another antimicrobial effect may center around fluoride's inhibition of polysaccharide synthesis, thereby causing an alteration of plaque composition.

The second mechanism suggested by which fluoride could be providing anticaries protection is in its interaction with tooth enamel. Fluoride and hydroxyapatite (HA) (major enamel component) form fluoroapatite (FA). When increased quantities of fluoride are available, it is postulated that the concentration of FA increases and the solubility of the original enamel is decreased (FA being less soluble than HA).

A third mechanism involves fluoride's ability, when retained in the oral environment following treatment, to accelerate the material remineralization of tooth enamel.

The prior art has addressed several ways in which to provide more fluoride to tooth enamel. For instance, topical high fluoride ion concentration treatments are provided by dentists. This requires a visit to the dentist and therefore methods and products for easier fluoride treatment to teeth have been and continue to be sought.

US-A-2,627,493 (Frederick G. Merckel, Jan. 18, 1955) teaches the use of a chewing gum which delivers fluoride to the teeth. US-A- 4,080,440 (David N. DiGiulio and R. J. Grabenstetter, March 21, 1978) typifies the use of a two-part remineralization product that delivers a fluoride source, a phosphate source and a calcium source to enamel. Another two-part product, such as that disclosed in US-A- 4,108,980 (Edward John Duff, August 22, 1978), involves the sequential application of a fluoride source followed by a calcium source. US-A-4,193,988 (Geoffrey C. Forward, et al, March 18, 1980) utilizes a two-part dentifrice (stripes of gel and toothpaste) to provide calcium ion and fluoride ion sources. However, none of the above references provide separate calcium and fluoride ion sources that allow for controlled $CaF_2$ precipitation. Prevention of precipitation is shown in US-A- 4,177,258 (Maria C. S. & Abdul Gaffar, December 4, 1979) and US-A-4,183,915 (Maria C. S. and Abdul Gaffar, January 15, 1980).

J. D. B. Featherstone, et al, Caries Research 15: 221-235 (1981) discloses compositions that may provide controlled precipitation of $CaF_2$ but not in the amounts contemplated herein and with fluoride levels below the limits of this invention.

References which disclose possible roles for $CaF_2$ in caries prevention are Marion D. Francis, et al, Adv. in Oral Biology 3: 83 - 120 (1968); H. G. McCann, Arch. Oral Biol. 13: 987 - 1001 (1968); Zao-Shon Liang and William I. Higuchi, J. Phys. Chem. 77: 1704 - 1710 (1973); V. Calavaska, et al, Arch. Oral Biol. 20: 333-339 (1975); M. Joost Larsen, et al, Scand. - Biol. 20: 327-333 (1977); S. Duke and G. C. Forward, Caries Res. 12: 12-20 (1978); M. Joost Larsen and O. Fejerskov, Scand. J. Dent. Res. 86: 337-345(1978); O. Fejerskov, et al, Acta Odont. Scand. 39:

241-249 (1981); S. Chandler, et al, J. Dent. Res. 61: 403-407 (1982). All of the above references are incorporated herein by reference.

The invention described herein provides anticaries protection via delivery of a controlled supersaturated $CaF_2$ solution to tooth enamel. A soluble calcium ion source, a calcium sequestering agent possessing specific solubility and binding properties and a soluble fluoride ion source are used in the present compositions. One execution of the present invention involves having all components present in a single composition. Other executions involve the use of the calcium ion source and the calcium sequestering agent in one composition and the fluoride ion source in another. When the composition(s) is used, it is desired to buffer the free calcium ion concentration such that an aqueous mixture with fluoride is near the critical supersaturation level for $CaF_2$. This will allow for maximum deposition of $CaF_2$. Below the critical level all of the components can be present together in a single aqueous composition (a metastable solution) while above the critical level two separate compositions are preferred. The essential as well as optional components are described in detail below.

The present invention provides anticaries protection by delivering controlled amounts of $CaF_2$ to tooth enamel. The invention uses a calcium-sequestering agent with a stability constant sufficient to inhibit uncontrolled and exceedingly rapid precipitation of $CaF_2$.

Calcium Ion Source

Calcium ion sources which can be used in the present invention are those sources safe for use in the oral cavity capable of providing at least about 0.015% total soluble calcium in ionic form and as soluble complexes of the sequestering agent when the product is used in the mouth. This is also the concentration in a single aqueous rinse composition product wherein all of the components are present. If the calcium ion source is present along with the sequestering agent in one aqueous rinse composition and the fluoride ion source is present in another, the amount of soluble calcium present when the two are mixed should be at least the 0.015%. Similarly in a dentifrice or other nonrinse product the concentration of soluble calcium when diluted in the mouth (generally a dilution of 1 part dentifrice to 3 parts saliva and water) is at least 0.015%. Using these data, the skilled artisan can easily make products having the proper concentration of components. If the composition(s) is an aqueous solution, and when diluted if it is not, it should have a pH from about 4.5 to about 8.8, preferably from about 6.5 to about 7.8. Preferably, calcium ion sources providing more than 5% total ionic and soluble complexed calcium in the mouth will not be used, and most preferred calcium ion sources should provide from about 0.04% to about 0.25% total soluble calcium in ionic and soluble complexed form. Representative sources include calcium hydroxide, calcium chloride, calcium acetate, calcium formate, calcium lactate, calcium nitrate, calcium gluconate, calcium glycerophosphate, calcium benzoate, calcium isobutyrate, calcium propionate, calcium salicylate, and mixtures thereof. Other suitable calcium ion sources include the calcium salts of the sequestering agents previously described.

The preferred calcium ion sources include the calcium salts of the sequestering agents, calcium hydroxide, calcium chloride, and mixtures thereof.

Calcium Sequestering Agents

Calcium sequestering agents with suitable stability constants, $K_m$, as defined by the following formula, are used to buffer the calcium ion concentration when the composition is used at levels such that the ion product of $[Ca] [F]^2$ is $\geq 2 \times 10^{-9} M^3$, or $mole^3/liter^3$.

$$K_m = \frac{[CaA]}{[Ca] [A]}$$

(In the above equation, [CaA] is the total molar concentration of the calcium complexed agent, [A] is the total molar concentration of sequestering agent not complexed by metal ion, and [Ca] represents the total molar concentration of uncomplexed calcium ion.)

It is seen that the ion product value, $[Ca][F]^2$, is also dependent on the free fluoride ion concentration. Therefore, the appropriate sequestering agent for use in such a product will depend on this fluoride concentration. It has been found that agents with effective stability constants ranging from $10^{2.8}$ to $10^6$ l/mole, preferably from about $10^{3.3}$ to about $10^{5.3}$ l/mole, provide the appropriate binding properties for calcium while maintaining appropriate levels of free fluoride ions.

Such agents must also be ones which do not reduce the total soluble calcium in ionic form and complexed form below 0.015% in the mouth. This is in order to provide enough calcium to react to form $CaF_2$. Sequestering agents present at molar concentrations from about 0.01 to about 0.10, provide optimal levels of calcium for calcium fluoride formation.

Examples of sequestering agents suitable for use in the present invention include N-(2-hydroxy-ethyl-iminodiacetic acid (supplied by Aldrich Chemical Company), nitrilotriacetic acid (supplied by Aldrich Chemical Company), N-(2-carboxyethyl)-iminodiacetic acid, DL-2-methylnitrilotriacetic acid, N-(phosphonomethyl)-iminodiacetic acid, N-(2-sulfoethyl)-iminodiacetic acid, DL-N-(2-hydroxyethyl)-2-methyl-iminodiacetic acid, N-(2-methoxyethyl)-iminodiacetic acid, N-(2-oxopropyl)-iminodiacetic acid, N-acetamidoiminodiacetic acid (supplied by Aldrich Chemical Company), N-hydroxyethylethylenediaminetriacetic acid (supplied by Aldrich Chemical Company), 2-aminobenzoic-N,N-diacetic acid, N,N-bis-(2-hydroxyethyl)-ethylenedinitrilo-N', N'diacetic acid, citric acid and mixtures thereof (supplied by Aldrich Chemical Company), or suitable salts thereof; for example, sodium, potassium, and calcium (Taken from Critical Stability Constants, A. E. Mortell and R. . Smith (Plenum Press: New York ) 1974 incorporated herein by reference.) Preferred sequestering agents are N-(2-hydroxyethyl-) iminodiacetic acid, N-hydroxyethyl-ethylenediaminetriacetic acid, N-acetaminodiacetic acid, nitrilotriacetic acid and mixtures thereof.

## Fluoride Ion Source

The present invention includes a fluoride ion source such that from about 0.0015% to about 0.5%, preferably from about 0.02% to about 0.10%, fluoride ion is provided when the product is used. The statements made earlier about the calcium ion concentration in the product composition(s) are also applicable to the fluoride ion source. Suitable fluoride sources include sodium fluoride, stannous fluoride, potassium fluoride, zinc fluoride, betaine fluoride, alanine stannous fluoride, sodium fluorosilicate, hexylamine fluoride, sodium monofluorophosphate and mixtures thereof. A preferred fluoride ion source is sodium fluoride. The fluoride that would be provided by sodium monofluorophosphate results from the hydrolysis monofluorophosphate undergoes to form fluoride and phosphate ions in aqueous solutions.

In order to provide the most effective anticaries benefit (minimal interference with fluoride deposition), phosphate ion levels should not exceed 0.06%, as the orthophosphate ion, when the present composition(s) is used.

When the present compositions providing enhanced anticaries protection are delivered to the teeth, at least 0.002 gram calcium ion, preferably from about 0.006 to about 0.030 gram calcium ion, should be provided by the calcium sequestering agent and calcium ion source component, while the amount of fluoride ion provided should be at least 0.0001 gram, preferably from about 0.003 to about 0.015 gram fluoride.

Examples of delivery systems utilizing two separate compositions in which the calcium ion source and sequestering agent are present in one composition and the fluoride ion source in another include the following: mouthwash-mouthwash; toothpaste-toothpaste; toothpaste-mouthwash; mouthwash-toothpaste; beverage-beverage; candy drop-candy drop; toothpowder-toothpowder; and so forth. While the above systems provide for physical separation of the compositions, the compositions may be used either simultaneously or sequentially, preferably simultaneously, i.e. a portion of the two mouthwashes mixed together just prior to use; a strip of each of the two toothpastes put onto a single brush; and so forth.

Other examples of systems which provide physical separation of the calcium ion materials and fluoride ions include, but are not limited to, a toothpaste in which one component is encapsulated for delayed release; a two-compartment bottle; a lozenge with a laminated structure; a laminated chewing gum; and so forth.

In addition to the systems described above wherein physical separation is provided, single compositions providing no separation may be used and discussed previously. Such a composition is a single phase mouthwash.

Suitable toothpastes can be made by using a 0.5% to 50%, preferably 5% to 35%, of an abrasive. Other ingredients include from about 0.2% to about 7% of a sudsing agent, from about 0.1% to about 5% of a binding agent, from 0% to about 50% of a humectant, with water and minors making up the balance.

Abrasives useful in the toothpastes include silica xerogels such as those disclosed in U.S. Patent 3,538,230, November 3, 1970, to Pader et al., incorporated herein by reference. Other toothpaste abrasives which can be used in the present invention are beta-phase calcium pyrophosphate, zirconium silicate, and thermosetting polymerized resins as described by Cooley et al. in U.S. Patent No. 3,070,510, December 25, 1962, incorporated herein by reference. In addition, precipitated silicas and insoluble metaphosphates can be used. Mixtures of abrasives are permitted with silica xerogels and precipitated silicas being preferred.

Suitable sudsing agents are preferably non-soap anionic or nonionic synthetic detergents. Water-soluble salts of alkyl sulfates having from about 10 to about 18 carbon atoms in the alkyl radical such as sodium lauryl sulfate are suitable. Other types of sudsing agents useful in this invention are water-soluble salts of sulfonated monoglycerides of fatty acids having from about 10 to about 18 carbon atoms of which sodium monoglyceride sulfonate is an example. Salts of $C_{10}$ - $C_{18}$ fatty acid amides of taurine are acceptable for use. Both sodium N-methyl-N-palmitoyl tauride and sodium N-coconut-acid-N-methyl taurate typify this category. Salts of $C_{10}$ - $C_{18}$ fatty acid esters of isethionic acid, and substantially saturated aliphatic acyl amides of saturated monoaminocarboxylic acids having 2 to 6 carbon atoms, and in which the acyl radical contains 12 to 16 carbon atoms are useful also. Sodium-N-lauryl sarcoside is an example of the latter.

A binding material is also useful in the present toothpaste compositions. One function the material serves is to thicken the product, while another function is to provide a desirable consistency for the toothpaste. Such ingredients include water-soluble salts of cellulose ethers such as sodium carboxymethyl cellulose, hydroxypropyl cellulose, and hydroxyethyl cellulose. Other thickeners which can be used are the natural gums which include gum karaya, gum arabic and gum tragacanth. Carboxyvinyl polymers (e.g. Carbopols®️ - B. F. Goodrich Co.) are also useful.

A humectant is desirably used to keep the toothpaste from hardening. Suitable humectants include glycerin, sorbitol, propylene glycol, and other edible polyhydric alcohols.

Flavoring agents are also useful in toothpastes. These flavoring agents include oil of wintergreen, oil of peppermint, oil of spearmint, oil of sassafras, and oil of close among many others. Along with a flavoring substance, sweeteners such as saccharin, dextrose, levulose, 6 methyloxathiazinone and sodium cyclamate can be used.

Additionally, cationic antimicrobial agents (e.g. bisbiguanides and quaternary ammonium compounds) such as those disclosed in U.S. Patent 4,183,916, January 15, 1980 to Rodon (incorporated herein by reference) may also be used in the present compositions. These agents preferably would be present in the fluoride composition if two compositions are employed.

The following examples, which are intended merely to illustrate the present invention and not be limiting thereof, set forth several types of suitable product forms. All percentages and ratios herein are by weight unless otherwise specified.

The following are two toothpastes representative of the present invention:

## EXAMPLE I

### CALCIUM ION SOURCE TOOTHPASTE

| INGREDIENT | % WEIGHT |
|---|---|
| Calcium N-(2-hydroxyethyl) iminodiacetate | 5.23 |
| N-(2-hydroxyethyl)iminodiacetic acid | 0.50 |
| Sorbitol (70% solution) | 41.17 |
| Precipitated silica | 20.00 |
| Sodium alkyl sulfate (28.8%) solution) | 4.00 |
| Carboxymethylcellulose | 1.50 |
| Xanthan gum | 0.50 |
| Distilled water | 25.00 |
| Flavor | 0.90 |
| Titanium dioxide | 0.70 |
| Sodium saccharin | 0.12 |
| *Sodium hydroxide (50% solution) | 0.38 |

*Adjust pH to between 7.75 - 7.85

## FLUORIDE ION SOURCE TOOTHPASTE

| INGREDIENT | % WEIGHT |
|---|---|
| Sodium fluoride | 0.485 |
| Sorbitol (70% solution) | 41.00 |
| Distilled water | 32.945 |
| Precipitated silica | 20.00 |
| Carboxymethylcellulose | 1.50 |
| Xanthan gum | 0.50 |
| Flavor | 0.80 |
| Sodium alkyl sulfate (28.8% solution) | 2.00 |
| Titanium dioxide | 0.70 |
| Sodium saccharin | 0.02 |
| Color | 0.05 |

*Adjust pH to between 7.0 - 7.2.

Another embodiment of the present invention is a two-part mouthwash. Mouthwashes typically contain from about 3% to about 60%, preferably 5% to 30%, ethyl alcohol. The alcohol acts as an antibacterial agent and as a solubilizer of other additives. Other components include from about 30% to about 90% water, from about 5% to about 20% of a humectant such as glycerin, from about 0.01% to about 0.1% antibacterial agent, from about 0.01% to about 0.5% sweetener, about 0.01% flavoring agent, and from about 0.1% to about 1% emulsifier such as polyoxyethylene (20) sorbitan mono-oleate. Those familiar with the art will recognize several flavoring agents and sweetening agents capable for use in a mouthwash product.

## EXAMPLE II

Two separate mouthwashes for use in accordance with the present invention are made of the following ingredients:

### CALCIUM ION SOURCE MOUTHWASH

| INGREDIENT | % WEIGHT |
|---|---|
| Calcium hydroxide | 0.28 |
| N-(2-hydroxyethyl)-iminodiacetic acid | .88 |
| Glycerin | 12.00 |
| Ethanol | 7.50 |
| Flavor | 0.06 |
| Sodium saccharin | 0.02 |
| Polysorbate 80* | 0.06 |
| Sodium benzoate | 0.155 |
| Distilled water | 79.045 |

*Polyoxyethylene(20)sorbitan mono-oleate

(Supplied by ICI Americas, Inc.)

Adjust pH to 7.8

### FLUORIDE ION SOURCE MOUTHWASH

| INGREDIENT | % WEIGHT |
|---|---|
| Sodium fluoride | 0.10 |
| Glycerin | 12.00 |
| Ethanol | 7.50 |
| Flavor | 0.06 |
| Sodium saccharin | 0.02 |
| Polysorbate 80* | 0.06 |
| Sodium benzoate | 0.155 |
| Distilled water | 80.105 |

*Polyoxyethylene(20)sorbitan mono-oleate

(Supplied by ICI Americas, Inc.)

Adjust pH to 7.0

### EXAMPLE III

A suitable single mouthwash for use in accordance with the present invention can be made in which a meaningful increase of fluoride uptake by tooth enamel is possible.

### SINGLE MOUTHWASH

| INGREDIENT | % WEIGHT |
|---|---|
| Sodium fluoride | 0.05 |
| Calcium hydroxide | 0.185 |
| N-hydroxyethylethylenediaminetriacetic acid | 0.720 |
| Glycerin | 12.00 |
| Ethanol | 7.50 |
| Flavor | 0.06 |
| Sodium saccharin | 0.02 |
| Polysorbate 80* | 0.06 |
| Sodium benzoate | 0.155 |
| Distilled water | 79.25 |

*Polyoxyethylene(20)sorbitan mono-oleate

(Supplied by ICI Americas, Inc.)

Adjust pH to 7.5

In addition to the above-discussed embodiments, the present invention can be practiced by using a calcium-containing toothpaste followed by a fluoride containing mouthrinse. The following example is such a suitable system for use in accordance with the present invention:

## EXAMPLE IV
### CALCIUM ION SOURCE TOOTHPASTE

| INGREDIENTS | % WEIGHT |
| --- | --- |
| Calcium hydroxide | 1.42 |
| N-(2-hydroxyethyl)iminodiacetic acid | 4.23 |
| Sorbitol (70% solution) | 39.43 |
| Precipitated silica | 20.00 |
| Sodium alkyl sulfate (28.8% solution) | 4.00 |
| Carboxymethylcellulose | 1.50 |
| Xanthan gum | 0.50 |
| Distilled water | 27.20 |
| Flavor | 0.90 |
| Titanium dioxide | 0.70 |
| Sodium saccharin | 0.12 |

*Sodium hydroxide (50% solution) to pH 7.8.

### FLUORIDE ION SOURCE MOUTHWASH

| INGREDIENTS | % WEIGHT |
| --- | --- |
| Sodium fluoride | 0.05 |
| Glycerin | 12.00 |
| Ethanol | 7.50 |
| Flavor | 0.06 |
| Sodium saccharin | 0.02 |
| Polysorbate 80* | 0.06 |
| Sodium benzoate | 0.155 |
| Distilled water | 80.155 |

*Polyoxyethylene(20)sorbitan mono-oleate

(Supplied by ICI Americas, Inc.)

Adjust pH to 7.0.

Another form of the present invention is as a chewing gum. Those familiar with the art will recognize that a gum base, humectant, sweeteners and flavors are the major ingredients of a gum. The following example is a chewing gum utilizing the essential components of the present invention:

## EXAMPLE V

### CALCIUM ION SOURCE

| INGREDIENTS | % WEIGHT |
|---|---|
| $Ca(OH)_2$ | 1.77 |
| N-Acetamidoiminodiacetic acid | 5.00 |
| Gum base | 25.00 |
| Sorbitol solution (70%) | 25.00 |
| Glycerin | 1.00 |
| Manitol powder | 40.63 |
| Flavor | 1.50 |
| Sodium saccharin | 0.10 |

### FLUORIDE ION SOURCE

| INGREDIENTS | % WEIGHT |
|---|---|
| Sodium fluoride solution (1%) | 2.0 |
| Gum base | 25.0 |
| Sorbitol solution (70%) | 25.0 |
| ·Manitol powder | 45.4 |
| Glycerin | 1.0 |
| Flavor | 1.5 |
| Sodium saccharin | 0.1 |

CLAIMS

1. An oral hygiene product providing anticaries protection to the teeth characterized by:

(A) a calcium ion source capable of providing in use at least 0.015% soluble calcium as defined in (B) below;

(B) a calcium sequestering agent or mixture of calcium sequestering agents effective in controlling the rate of precipitation of $CaF_2$, said sequestering agent being one that does not reduce the total soluble calcium, said total soluble calcium comprising both ionic calcium and calcium complexed by the sequestering agent, below 0.015%, and being one with an effective stability constant ($K_m$) ranging from $10^{2.8}$ to $10^6$ 1/mole, as defined by the followng equation:

$$K_m = \frac{[CaA]}{[Ca] \ [A]}$$

wherein [Ca] is the total molar concentration of uncomplexed calcium ion, [A] is the total molar concentration of soluble sequestering agent uncomplexed by any metal ions, and [CaA] is the total molar concentration of soluble sequestering agent complexed by calcium; and

(C) a fluoride ion source capable of providing in use from 0.0015% to 0.5% fluoride, wherein said product has a pH of from 4.5 to 8.8 at 25°C - 37°C if an aqueous solution or when diluted with salivary secretions.

2. An oral hygiene product according to Claim 1, characterized in that the calcium sequestering agent or mixture of calcium sequestering agents has an effective stability constant ($K_m$) ranging from $10^{3.3}$ to $10^{5.3}$ 1/mole.

- 2 -

**0089136**

3. An oral hygiene product according to Claim 1 or 2, characterised in that the calcium sequestering agent is selected from substituted iminodiacetic acids, substituted iminotriacetic acids, citric acid, the sodium, potassium, and calcium salts of these sequestering agents and mixtures thereof.

4. An oral hygiene product according to Claim 3, characterized in that the calcium sequestering agent(s) is selected from N-(2-hydroxyethyl)-iminodiacetic acid, nitrilotriacetic acid, N-(2-carboxyethyl)-iminodiacetic acid, DL-2-methylnitrilotriacetic acid, N-(phosphonomethyl)-iminodiacetic acid, N-(2-sulfoethyl)-iminodiacetic acid, DL-N-(2-hydroxyethyl)-2-methyliminodiacetic acid, N-(2-methoxyethyl)-iminodiacetic acid, N-(2-oxopropyl)-iminodiacetic acid, N-acetamidoiminodiacetic acid, N-hydroxyethylethylenediaminetriacetic acid, 2-aminobenzoic-N,N-diacetic acid, N,N-bis-(2-hydroxyethyl)-ethylenedinitrilo-N', N'-diacetic acid, citric acid, sodium potassium, and calcium salts of these sequestering agents, and mixtures thereof.

5. An oral hygiene product according to Claim 4, characterized in that the calcium ion source is selected from calcium hydroxide, calcium chloride, calcium acetate, calcium formate, calcium lactate, calcium nitrate, calcium gluconate, calcium glycerophosphate, calcium benzoate, calcium isobutyrate, calcium propionate, calcium salicylate, the calcium salts of the sequestering agents, and mixtures thereof.

6. An oral hygiene product according to any of claims 1 to 5 characterized in that the fluoride ion source is capable of providing from 0.02% to 0.10% fluoride ion and is selected from sodium fluoride, stannous fluoride, potassium fluoride, zinc fluoride, betaine fluoride, alanine stannous fluoride, sodium fluorosilicate, hexylamine fluoride, sodium monofluorophosphate, and mixtures thereof.

7. An oral hygiene product according to any of Claims 1 to 6 which is in the form of a single mouthwash composition.

8. An oral hygiene product according to any of Claims 1 to 6 characterized in that said product is in the form of two compositions wherein one composition contains the calcium sequestering agent and the calcium ion source and the other composition contains the fluoride ion source, both compositions have a pH of from 4.5 to 8.8 if aqueous solutions or when diluted with salivary excretions.

9. An oral hygiene product according to Claim 8 characterized by a delivery system providing for physical separation of the two compositions and for simultaneous or sequential application thereof to teeth.